# FASCICULE DE BREVET EUROPEEN

(11) **EP 3 126 318 B1**
(45) Date de publication et mention de la délivrance du brevet: **21.03.2018**
(21) Numéro de dépôt: 15732019.3
(22) Date de dépôt: 26.03.2015
(51) Int. Cl.: C07C 45/37, C07C 47/22

(54) **PROCÉDÉ DE SYNTHÈSE DIRECTE DE (MÉTH)ACROLÉINE A PARTIR D'ÉTHERS ET/OU ACÉTALS**
VERFAHREN ZUR DIREKTEN SYNTHESE VON (METH)ACROLEIN AUS ETHERN UND/ODER ACETALEN
PROCESS FOR DIRECT SYNTHESIS OF (METH)ACROLEIN FROM ETHERS AND/OR ACETALS

(30) Priorité: 04.04.2014 FR 1453022
(43) Date de publication de la demande: 08.02.2017
(73) Titulaire: Arkema France, 92700 Colombes (FR)
(72) Inventeur: DUBOIS, Jean-Luc, F-69390 Millery (FR)
(74) Mandataire: Bonnel, Claudine
(86) Numéro de dépôt international: PCT/FR2015/050769
(87) Numéro de publication internationale: WO 2015/150666

(56) Documents cités:
- GB-A- 513 772
- US-A- 4 433 174

## Description

### Domaine technique

La présente invention concerne la synthèse d'aldéhydes insaturés. Elle a plus particulièrement pour objet un procédé de synthèse directe de (méth)acroléine à partir d'un mélange réactif comprenant au moins un composé choisi parmi les éthers, acétals ou hémiacétals dérivés d'alcools linéaires comprenant de 1 à 3 atomes de carbone.

### Arrière-plan technique et problème technique

Le terme « (méth)acroléine» signifie de façon conventionnelle acroléine ou méthacroléine, de formule CH₂=CH-CHO ou CH₂=CH(CH₃)-CHO respectivement.

Les grands procédés industriels connus de fabrication de l'acroléine sont basés sur différents schémas réactionnels mettant en oeuvre des matières premières différentes.

Les procédés mettant en oeuvre une réaction d'aldolisation, tels que décrits par exemple dans le brevet UK 513,772 de Degussa, utilisent comme matières premières l'acétaldéhyde et le formol qui réagissent en phase gazeuse en catalyse hétérogène pour former l'acroléine. Le formol et l'acétaldéhyde ne se stockent ni ne se transportent facilement ; par exemple, le formol peut être transporté uniquement sur un rayon de 400 km autour du site de production, et doit pour cela être stabilisé avec du méthanol en solution aqueuse. Pour s'affranchir de l'achat des matières premières, il est nécessaire de prévoir des unités spécifiques de synthèse de ces produits qui s'ajoutent au coût de l'unité d'aldolisation proprement dite. La synthèse de l'acétaldéhyde est généralement réalisée par la réaction de Waker d'oxydation de l'éthylène et celle du formol par oxydation (ou oxydéshydrogénation selon le système catalytique retenu) du méthanol. Le procédé de synthèse de l'acroléine selon la voie aldolisation nécessite donc des investissements importants.

Les procédés industriels fondés sur l'oxydation d'oléfines, en particulier l'oxydation du propylène conduisant à l'acroléine, qui a fait l'objet de nombreuses publications ou brevets, consomment des matières premières fossiles, extraites du pétrole notamment, dont le coût d'accès devient de plus en plus élevé. Par ailleurs, leur disponibilité est liée à des installations de raffinage ou de pétrochimie de grandes capacités ce qui impose en pratique une implantation à proximité des grands sites pétrochimiques, et donc des coûts de transport vers le consommateur final sans compter les risques d'émissions de produits polluants.

Plus récemment, une nouvelle voie de synthèse basée sur la réaction de déshydratation du glycérol en acroléine, a fait l'objet de travaux importants, notamment par la Demanderesse (WO 2006/087083 ; WO 2006/087084 ; WO 2009/128555 ; WO 10/046227; WO 11/083225). Ce type de procédé présente l'avantage de pouvoir travailler avec une matière première naturelle renouvelable (non fossile) et donc de répondre aux engagements de la plupart des pays industrialisés visant à réduire les émissions de gaz à effet de serre avec ses effets environnementaux, mais aussi de fournir une solution de production plus durable.

Cependant, cette voie de synthèse présente certains désavantages sur plusieurs plans. En effet, l'approvisionnement en glycérol dépend principalement des unités oléo chimiques et/ou de biodiésel. Le glycérol n'est disponible qu'en quantités limitées ce qui restreint en pratique la taille possible des unités de production d'acroléine et donc leur rentabilité économique. Les qualités de glycérol les plus répandues sont dites de Glycérine brute, ce qui correspond le plus souvent à des solutions aqueuses de glycérol à 80 % poids contenant des sels (par exemple NaCl, KCl, Na₂SO₄, K₂SO₄...), du méthanol résiduel quand il a été obtenu par méthanolyse des huiles végétales, des Matières Organiques Non Glycérineuses (MONG), et toutes sortes d'impuretés extraites des plantes ou graisses animales lors des procédés de production. Ces impuretés sont des poisons pour les catalyseurs et conduisent à une désactivation réversible, par exemple par formation de coke, ou irréversible, par exemple par dépôt des sels. De la glycérine raffinée est aussi disponible commercialement, mais à des prix beaucoup plus élevés. Sur un plan technique, les catalyseurs de déshydratation connus jusqu'à maintenant se désactivent rapidement ce qui nécessite des régénérations régulières impliquant un surcoût en investissement (par exemple un doublement ou triplement des volumes catalytiques). Enfin, ce procédé « vert » présente un inconvénient du fait que la réaction de déshydratation, effectuée en phase gaz, globalement forte consommatrice d'énergie est dépendante du coût de cette énergie aujourd'hui fournie à partir de combustibles fossiles ayant aussi obligatoirement un impact environnemental.

Par ailleurs, des alcools, en particulier un mélange de méthanol et d'éthanol, ont déjà été décrits comme matières premières pour former de l'acroléine. On peut citer par exemple la demande WO 2005/040392 qui décrit l'oxydation d'un mélange de méthanol et d'éthanol en présence d'un catalyseur à l'argent, conduisant à un mélange d'acétaldéhyde et de formaldéhyde, qui est ensuite converti en acroléine en présence d'alumine. L'exemple 32 de ce document utilise un mélange réactionnel dont la composition n'est pas précisée, et aucune indication sur le rendement du procédé n'est mentionnée. Ce procédé présente l'inconvénient de devoir adapter la température de réaction à chacune des étapes.

En ce qui concerne la synthèse de la méthacroléine, les procédés industriels utilisent généralement comme matières premières l'isobutane, le tertiobutanol ou l'isobutène qui sont transformés en méthacroléine par oxydation. Il existe d'autres procédés de synthèse tels que l'oxydéshydrogénation de l'isobutaraldéhyde ou l'hydroformylation du méthylacétylène ou du propadiène. Outre dans certains cas leur coût, l'origine fossile de ces matières premières est un désavantage majeur.

La méthacroléine peut être obtenue aussi par réaction d'aldolisation, en particulier entre le formaldéhyde et le propionaldéhyde selon le procédé décrit dans le brevet US 4,433,174, avec cependant les inconvénients liés à l'utilisation de ces aldéhydes.

Il subsiste donc un réel besoin de pallier les inconvénients des procédés de synthèse de (méth)acroléine de l'art antérieur, notamment de réduire leur impact environnemental et de limiter leurs investissements ou leurs coûts de fonctionnement.

Un des objectifs de la présente invention est donc de fournir un procédé de synthèse de (méth)acroléine, utilisant des matières premières d'origine renouvelable (non fossiles) qui soit simple, rapide (comportant le moins d'étapes possibles), facile à mettre en oeuvre et qui conduise au produit recherché avec une bonne sélectivité .

Les inventeurs ont découvert qu'il était possible de synthétiser l'acroléine et la méthacroléine par couplage d'une réaction d'oxydation et d'une réaction d'aldolisation, en utilisant un mélange réactif comprenant au moins un composé choisi parmi les éthers, acétals ou hémiacétals dérivés d'alcools linéaires comprenant de 1 à 3 atomes de carbone, dont certains sont des composés disponibles à partir de matières premières renouvelables.

### Résumé de l'invention

L'invention a donc pour objet un procédé de synthèse directe de (méth)acroléine comprenant i) la réaction d'un mélange réactif comprenant au moins un composé réactif, de l'oxygène et un gaz diluant non réactif, dans un ensemble réactionnel opéré en phase gazeuse à une température comprise entre 200°C et 400°C et sous une pression comprise entre 1 et 10 bars, en présence d'un catalyseur d'oxydation solide choisi parmi les catalyseurs à base de molybdène et éventuellement d'un catalyseur de condensation par aldolisation, puis ii) la récupération de l'effluent gazeux comprenant la (méth)acroléine formée en présence d'eau coproduite par la réaction,
ledit procédé étant caractérisé
- en ce que le(s) composé(s) réactif(s) est (sont) choisi(s) parmi les composés de formule (I) R₁-O-R₂ dans laquelle,
   R₁ est H, ou un radical méthyl, éthyl ou propyl,
   R₂ est un radical méthyl, éthyl, propyl ou un radical de formule CH₂-O-R₃, CH(CH₃)-OR₃, CH(CH₂-CH₃)-O-R₃, avec R₃ est un radical méthyl, éthyl ou propyl,
   avec R₁ et R₂ identiques ou différents, ou R₁ et R₃ identiques ou différents,
   étant entendu que lorsque R₁ est H, R₂ est différent d'un radical méthyl, éthyl, propyl, à défaut de quoi le mélange réactif comprend au moins un autre composé de formule (I) avec R₁ différent de H,
- et en ce que le(s) composé(s) réactif(s) est (sont) choisi(s) de sorte que les radicaux R₁, R₂ et R₃ présents dans leur structure soient aptes à former de la (méth)acroléine par réaction d'oxydation couplée à une réaction d'aldolisation.

Au sens de l'invention, « procédé de synthèse directe », signifie que la synthèse s'effectue en une étape sans isolation de composés intermédiaires. Ce procédé est conduit dans un seul ensemble réactionnel qui comporte, soit un réacteur unique, soit deux réacteurs successifs mais dans ce cas, le second réacteur est alimenté par l'effluent de sortie du premier réacteur.

Le procédé de l'invention est particulièrement avantageux puisqu'il est mis en oeuvre avec des composés réactifs contenant déjà une forme oxydée par rapport aux alcools correspondants utilisés comme réactifs. Le choix particulier du composé réactif ou de la combinaison des composés réactifs permet de limiter la quantité d'eau générée par la réaction, de limiter les réactions inverses ou les réactions de dégradation, et d'obtenir ainsi le produit recherché plus concentré. De plus, le procédé est conduit dans un seul ensemble réactionnel, et peut être effectué en présence d'un catalyseur unique, notamment en présence d'un catalyseur d'oxydation à base de molybdène, simplifiant ainsi les conditions opératoires de mise en oeuvre du procédé.

L'invention est maintenant décrite plus en détail et de façon non limitative dans la description qui suit.

### Description détaillée de l'invention

Selon l'invention, on utilise comme matière première pour produire l'acroléine ou la méthacroléine, au moins un composé réactif choisi parmi les éthers, acétals ou hémiacétals dérivés d'alcools linéaires comprenant de 1 à 3 atomes de carbone, seul ou éventuellement en combinaison avec un alcool linéaire comprenant de 1 à 3 atomes de carbone.

Le composé réactif répond à la formule générale (I) R₁-O-R₂, R₁, R₂ et R₃ ayant les significations précitées, et lorsque la formule correspond à un alcool (R₁ est l'atome d'hydrogène et R₂ est un radical méthyl, éthyl, propyl), alors au moins un autre composé de formule (I) qui n'est pas un alcool est utilisé en association.

Comme exemples de composés réactifs utilisables, on peut citer sans que cette liste soit limitative, le diméthyl éther (CAS 115-10-6 - BP=-24,8°C) ; le diéthyl éther (CAS 60-29-7 - BP=34,6°C) ; le méthyl éthyl éther (CAS 540-67-0 - BP=7,4°C) ; le diméthoxy méthane (CAS 109-87-5 - BP= 42°C) ; le diéthoxy méthane (CAS 462-95-3 - BP= 88°C) ; le dipropoxy méthane (CAS 505-84-0 - BP=137,6°C) ; le 1,1-diméthoxy éthane (CAS 534-15-6 - BP= 64°C) ; le 1,1-diéthoxy éthane (CAS 105-57-7-BP=103°C).

Comme composés réactifs préférés, on peut citer le diméthyl éther (DME), le diéthyl éther (DEE), le diméthoxy méthane (DMM), le méthyl éthyl éther (MEE), le 1,1-diméthoxy éthane (DMEt), et le diéthoxyméthane (DEM).

Ces composés sont disponibles commercialement, ou peuvent être produits selon des voies mettant en oeuvre des matières renouvelables. Par exemple, le diméthyl éther (DME), tout comme le méthanol, peut être produit directement à partir de gaz de synthèse. Le diméthyl éther est donc une matière première disponible en grande quantité et à bas prix. C'est aussi un intermédiaire dans les procédés dits « Methanol to Propylene ». Par ailleurs, il s'agit d'un bien meilleur carburant que le méthanol.

L'utilisation de composés réactifs de type éther, acétal ou hémiacétal, du fait de leur structure (il y a 2 carbones dans le DME pour 1 carbone dans le méthanol), entraine une diminution du flux molaire et donc du flux volumique, par rapport à l'utilisation des alcools correspondants. Il en résulte des avantages notamment sur la perte de charge, et la consommation en énergie de compression dans le procédé de l'invention.

Avantageusement, on utilise comme composés réactifs de formule (I) les composés gazeux à température ambiante, tel que le diméthyl éther (DME), ou les composés ayant un point d'ébullition bas, tels que par exemple le diéthyl éther (DEE) ou le diméthoxy méthane (DMM). Ces réactifs nécessitent des températures plus basses pour être vaporisés, voire sont déjà gazeux, ce qui permet d'utiliser des flux résiduaires à bas niveaux thermiques pour la vaporisation des charges réactionnelles et facilite leur utilisation dans le procédé de l'invention. Les flux résiduaires à bas niveaux thermiques sont par exemple les flux issus d'étapes de condensation sur les colonnes à distiller. Il s'agit en général de flux disponibles sur les sites industriels en grande quantité, et à des niveaux thermiques inférieurs à 140 °C et de préférence inférieurs à 110 °C, et encore plus préféré inférieurs à 90 °C. Il s'agit par exemple de vapeur d'eau condensée.

La réaction mise en oeuvre dans le procédé selon l'invention est basée sur une première phase d'oxydation d'un (ou de) composé(s) réactif(s) de type éther, acétal ou hémiacétal, éventuellement combiné avec un alcool, conduisant à la formation d'aldéhydes, qui sont ensuite transformés par réaction d'aldolisation en acroléine ou (méth)acroléine.

Le(s) composé(s) réactif(s) est (sont) donc choisi(s) de sorte que les radicaux R₁, R₂ et R₃ présents dans leur structure soient aptes à former de la (méth)acroléine par réaction d'oxydation couplée à une réaction d'aldolisation.

Ainsi lorsqu'un réactif (I) unique est utilisé, pour la formation d'acroléine, alors R₁, R₂ ou R₃ est un radical méthyl ou méthylène, et au moins l'un des autres radicaux est un radical CH₃-CH₂ ou CH₃-CH. Pour la formation de méthacroléine, R₁, R₂ ou R₃ est un radical méthyl ou méthylène, et au moins l'un des autres radicaux est un radical CH₃-CH₂-CH₂ ou CH₃-CH₂-CH.

Lorsqu'un mélange de réactifs est utilisé par exemple, pour former de l'acroléine, on peut utiliser un composé de formule (I) comportant au moins un radical méthyl ou méthylène (CH₂-) et un autre composé de formule (I) comportant au moins un radical CH₃-CH₂ ou CH₃-CH.

Pour former de la méthacroléine, on peut utiliser un composé de formule (I) comportant au moins un radical propyl et un composé de formule (I) comportant au moins un radical méthyl ou méthylène, ou un composé mixte comportant un radical propyl ou CH₃-CH₂-CH et un radical méthyl ou méthylène.

Comme exemples de composés réactifs ou d'associations de composés réactifs conduisant à l'acroléine, on peut citer diméthyl éther / diéthyl éther ; méthyl éthyl éther ; diméthyl éther / diéthoxy méthane ; diéthyl éther / diméthoxy méthane ; diméthoxy méthane / diéthoxy méthane ; diméthoxy méthane / diéthoxy éthane ; diméthyl éther / éthanol ; diéthyl éther / méthanol ; diméthoxy méthane / éthanol.

Comme exemples de composés réactifs ou d'associations de composés réactifs conduisant à la méthacroléine, on peut citer le dipropoxyméthane, le dipropyl éther / méthanol ; le dipropyl éther / diméthyl éther.

Le procédé selon l'invention peut par exemple être illustré par les schémas réactionnels suivants :

CH₃-O-CH₃ + CH₃-CH₂-O-CH₂-CH₃ + 2 O₂ → 2 CH₂=CH-CHO + 4 H₂O

CH₂(O-CH₃)₂ + CH₃-CH₂-O-CH(CH₃)-OCH₂-CH₃ + 2 O₂ → 3 CH₂=CH-CHO + 5 H₂O

2 CH₂(O-CH₃)₂ + 3 CH₃-CH₂-O-CH₂-CH₃ + 5 O₂ → 6 CH₂=CH-CHO + 11 H₂O

CH₂(O-CH₂-CH₂-CH₃)₂ + x O₂ → CH₂=C(CH₃)-CHO + y H₂O + produits secondaires

Les ratios molaires, calculés sur la base des radicaux R₁, R₂ et R₃ des composés réactifs mis en oeuvre, par exemple,
(CH₃+CH₂)/(CH₃-CH₂ + CH₃-CH) d'une part, ou (CH₃+CH₂)/(CH₃-CH₂-CH₂+CH₃-CH₂-CH) d'autre part, sont compris entre 1 et 2 et de préférence entre 1,1 et 1,5.

Les schémas réactionnels mis en oeuvre dans le procédé selon l'invention nécessitent moins d'oxygène et génèrent une quantité d'eau moindre comparativement à la mise en oeuvre d'un mélange d'alcools selon les réactions suivantes :

CH₃-OH + CH₃-CH₂-OH + O₂ → CH₂=CH-CHO + 3 H₂O

CH₃-OH + CH₃-CH₂-CH₂-OH + O₂ → CH₂=C(CH₃)-CHO + 3 H₂O

L'effluent gazeux comprenant la (méth)acroléine formée en présence de l'eau coproduite par la réaction est ainsi plus concentré.

Outre le(s) composé(s) réactif(s) de formule (I), le mélange réactif introduit dans l'ensemble réactionnel comprend de l'oxygène et un gaz diluant non réactif.

Le débit d'introduction des composés réactifs dans l'ensemble réactionnel est tel que leur teneur totale dans le mélange réactif est comprise entre 1% et 15%, de préférence entre 2% et 12%, et de manière plus préférée entre 2 et 6 % exprimée en volume.

Le débit d'introduction de l'oxygène sera tel que la teneur en oxygène du mélange réactif ne sera pas supérieure à 15%, et de préférence inférieure à 10% exprimée en volume. La teneur en oxygène du mélange est de préférence d'au moins 1 % vol, et de préférence d'au moins 5 % vol.

Néanmoins, sous réserve d'équiper le réacteur de disques de ruptures ou autres équipements de sécurité, il est possible de fonctionner avec des concentrations en oxygène plus élevées.

Le reste du mélange réactif est constitué d'un ou plusieurs gaz inertes qui représentent de l'ordre de 70% à 95% en volume du milieu réactionnel. La présence de ces derniers est indispensable pour éviter que le mélange ne soit situé dans la zone d'explosion. Ces gaz inertes seront par exemple, du gaz carbonique, de l'azote ou un gaz rare tel que l'argon ou l'hélium. Il peut encore s'agir d'hydrocarbures tel que le méthane qui est inerte dans les conditions de réaction.

Les réactions dans le système réactionnel sont conduites avec une VVH (vitesse volumique horaire) généralement comprise entre 2 000 et 40 000h⁻¹ et de préférence entre 5 000 et 25 000h⁻¹, la VVH représentant le ratio entre le débit de gaz total (en normaux-litres) divisé par le volume de catalyseur (masse volumique apparente prise à 1 g/ml).

Dans le procédé de l'invention, on utilise un catalyseur d'oxydation solide choisi parmi les catalyseurs à base de molybdène et éventuellement un catalyseur de condensation par aldolisation.

Les catalyseurs d'oxydation sont bien connus depuis des décennies. Dans le procédé de l'invention, on utilise un catalyseur solide d'oxydation de type oxyde mixte à base de molybdène.

Les catalyseurs d'oxydation utilisables dans le procédé de l'invention comprennent le molybdène et au moins un élément choisi parmi P, Si, W, Ti, V, Nb, Ta, Cr, Mn, Fe, Co, Ni, Cu, Zn, Ga, In, Tl, Sb, Ag, As, Ge, B, Bi, La, Ba, Sb, Te, Ce, Pb, choisis dans le groupe constitué par les oxydes mixtes contenant le molybdène et les hétéropolyacides contenant le molybdène.

Ces catalyseurs peuvent être représentés par la formule générale suivante.

**A**ₐ **X**_{b} **Mo**_{c} **Z**_{d} **O**ₑ

dans laquelle
**A** est au moins un cation choisi parmi les éléments des Groupes 1 à 16 de la Classification Périodique des Eléments et les lanthanides, de préférence un cation d'un métal alcalin tel que Cs, Rb ou K,
**X** est P ou Si, et de préférence P
**Z** est au moins un élément choisi dans le groupe comprenant par W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce and Pb et de préférence Cu, Fe, Bi, Co, Ni, W, V, Cr, Sb, Mn, Ce,
**O** est l'oxygène
a, b, c et d sont des indices constitués de nombres entiers ou décimaux satisfaisant aux plages suivantes
0 ≤ a ≤ 9 et de préférence 0 < a ≤ 9
0 ≤ b ≤ 2 et de préférence 0,1 ≤ b ≤ 1,5
0 ≤ c ≤ 12 et de préférence 5 < c ≤ 12
0 ≤ d ≤ 12 et de préférence 0 < d ≤ 4
tels que a + b + d > 0
et e est un nombre déterminé par le degré total d'oxydation des éléments.

Le catalyseur utilisé lors de l'étape d'oxydation peut être un catalyseur du type Fe-Mo-O dopé ou non, utilisé généralement lors de l'oxydation du méthanol en formaldéhyde. Des exemples de catalyseurs molybdate de fer (Fe-Mo-O) sont décrits dans Catalysis Review, Vol 47 (2005), pp 125-174*.*

Ces catalyseurs sont disponibles commercialement. Par exemple Mapco, fournit un catalyseur avec sa licence de technologie et Süd Chemie fournit plusieurs grades de ces catalyseurs sous la marque FAMAX® : FAMAX J5, FAMAX MS, FAMAX HS, FAMAX TH. Dans le cas de ce type de catalyseur Fe-Mo, les indices a et b de la formule générale ci-dessus seront de préférence de valeur 0 et l'indice d sera de valeur non nulle.

De préférence, notamment dans la variante du procédé utilisant un catalyseur unique, le catalyseur d'oxydation est un catalyseur de type molybdate de fer.

Les catalyseurs d'oxydation peuvent être sous forme massique et utilisés dans ce cas sans support.

Les catalyseurs peuvent aussi être déposés sur un support inactif dont la quantité représente de 30 à 90% et de préférence au moins 50% du poids total du catalyseur.

Il est possible d'utiliser comme support tout matériau tel que la stéatite, la silice, l'alumine, la magnésie, l'oxyde de titane, la zircone, le carbure de silicium, les silicates, les terres de diatomées, les borates ou carbonates, des céramiques, à condition que les matériaux soient stables aux conditions opératoires auxquelles les catalyseurs sont soumis.

Le catalyseur massique ou le catalyseur supporté peuvent être sous forme de granulé ou de poudre et se présenter sous une forme quelconque telle que sphère, grain, cylindre creux, trilobe et quadrilobe, ainsi que sous la forme de cylindres, extrudés ou compressés éventuellement en utilisant un agent de pastillage. De préférence le catalyseur est sous la forme de cylindres creux ou de trilobes creux.

Dans le procédé de l'invention, on peut utiliser en association avec le catalyseur d'oxydation, un catalyseur solide de condensation par aldolisation, par exemple parmi ceux connus pour réaliser la condensation de deux aldéhydes et notamment du formol avec un aldéhyde supérieur (acétaldéhyde ou propanaldéhyde), pour conduire à l'aldéhyde insaturé correspondant en deux étapes : réaction d'aldolisation du formaldéhyde sur l'aldéhyde supérieur avec formation d'un aldéhyde hydroxylé (aldol) puis réaction de déshydratation de cet aldol.

Les catalyseurs de condensation utilisés dans le procédé de l'invention appartiennent aux différentes catégories suivantes.
1) les «bases supportées », c'est-à-dire les hydroxydes alcalins LiOH, NaOH, KOH, CsOH déposés sur support silice ou alumine, les métaux alcalins et alcalinoterreux dispersés sur silice, alumine (Al₂O₃-NaOH-Na), magnésie (MgO-NaOH), charbon ou carbonate de potassium, les composés azotés, NR₃, NH₃, KNH₂ déposés sur alumine, LiCO₃ déposé sur silice, t-BuOK déposé sur xonotlite et plus généralement les solides de type métaux alcalins déposés sur alumine (tels que Na/Al₂O₃ ou KF/Al₂O₃), sur silice ou sur magnésie (tel que Li/MgO).
   Ce catalyseur solide est constitué par exemple de silicate de sodium déposé sur silice ou sur aluminosilicate présentant de préférence un rapport atomique Si/Al supérieur à 10 et comportant le cas échéant un promoteur métallique ou encore du césium déposé sur une silice greffée ou dopée par un composé du zirconium (Cs-Zr/SiO₂).
2) les « oxydes métalliques » BaO, BeO, SrO, CaO, Al₂O₃, Y₂O₃, La₂O₃, CeO₂, ThO₂, SnO₂, K₂O, Na₂O, MgO, ZnO, TiO₂, ZrO₂ sous forme oxyde ou carbonate éventuellement dopés avec un métal alcalin, les oxydes ou oxycarbonates de terres rares éventuellement dopés par des alcalins.
3) Les « sels métalliques » des composés ci-dessus, c'est-à-dire les carbonates, hydroxycarbonates, hydrogénocarbonates, sels d'ammonium, etc.
4) Les « oxydes mixtes » tels que SiO₂-MgO, SiO₂-CaO_{,} SiO₂-SrO, SiO₂-BaO, SiO₂-SnO, SiO₂-ZnO, SiO₂-Al₂O₃, SiO₂-ThO₂, SiO₂-TiO₂, SiO₂-ZrO₂, SiO₂-MoO₃, SiO₂-WO₃, Al₂O₃-MgO, Al₂O₃-ThO₂, Al₂O₃-TiO₂, Al₂O₃-ZrO₂, Al₂O₃-MoO₃, Al₂O₃-WO₃, ZrO₂-ZnO, ZrO₂-TiO₂, TiO₂-MgO, ZrO₂-SnO₂. A cette liste d'oxydes mixtes, on peut ajouter les oxydes de type argile comme les hydrotalcites, hydroxyapatites, chrysolite et sépiolite, éventuellement dopés par des alcalins, ainsi que par des métaux tels que le cuivre, le fer, le nickel, ou les oxydes de terres rares dopés par des alcalino-terreux tels que (SrO-La₂O₃). Relèvent également de cette catégorie, d'autres catalyseurs qui peuvent aussi convenir pour cette réaction tels que les catalyseurs oxydes mixtes de type phosphates mixtes de cobalt et aluminium, ou silice-alumine dopées par exemple avec des sels de sodium (Na), potassium (K), césium (Cs), cadmium (Cd), Mg, Ca, Sr, Mn, Zn, Mo, Nb, Pb et/ou Si. Ils pourront être aussi MgO-alumine, MgO-SiO₂, des terres rares, sous forme de phosphates, tungstates, molybdates, etc. Les oxynitrures de dérivés phosphorés tels que les oxynitrures mixtes de Vanadium-Aluminium, Phosphore-Zirconium, Phosphore-Aluminium, Vanadium-Aluminium-Phosphore, Gallium-Aluminium-Phosphore peuvent aussi convenir pour cette réaction.
5) Différentes « zéolithes » échangées avec des ions alcalins (Cs, K, Na, Li).

On peut citer à titre d'exemple de tels catalyseurs, les aluminosilicates cristallisés ou amorphes, les silicalites, les zéolithes synthétiques cristallines telles que la Faujasite, la Ferriérite, la ZSM-5, sous leur forme acide ou sous une forme soit partiellement, soit totalement neutralisée par des éléments des groupes 1 à 14, et de préférence des groupes 1 et 2 et par Zn et Tl. Les zéolithes utilisées peuvent avoir dans leur structure une partie ou la totalité des atomes d'aluminium remplacés par des atomes trivalents tels que B, Ga, Fe, Cr, V, As, Sb, Bi, Be, et peuvent avoir une partie ou la totalité des atomes de silicium remplacés par des atomes tétravalents tels que Ge, Ti, Zr, Hf.

Les catalyseurs solides de condensation utilisés dans le procédé selon l'invention présentent une surface spécifique élevée, généralement comprise entre 40 et 600 m²/g et de préférence entre 50 et 200 m²/g.

Les propriétés acide-base d'un matériau peuvent être liées à sa composition par exemple, sa structure cristalline ou à des défauts de surface tels la présence d'impuretés comme les métaux alcalins ou alcalino-terreux.

La présence de sites basiques et leurs quantités peuvent être déterminées par toute méthode connue, par exemple par adsorption d'un composé acide tel que CO₂ ou SO₂ et par des mesures micro-calorimétriques de l'énergie d'adsorption. La présence de sites acides et leurs quantités peuvent quant à elles être mesurées par adsorption d'un composé basique tel que l'ammoniac par exemple.

Les mesures de l'acidité et de la basicité du catalyseur sont réalisées par adsorption de SO₂ (basicité) et de NH₃ (acidité) et par les mesures micro-calorimétriques de l'énergie d'adsorption. Les conditions opératoires habituelles sont les suivantes.

Les essais sont menés à 150°C dans un calorimètre (C80 de Setaram) relié à un appareil volumétrique conventionnel équipé d'un manomètre capacitif Barocel pour les mesures de pression. Les échantillons sont prétraités dans une cellule en quartz par chauffage pendant une nuit sous vide à 300°C. Cette température est atteinte par augmentation de température au rythme de 1°C/min. Les chaleurs différentielles d'adsorption sont mesurées comme en fonction du taux de recouvrement par envoi répétitif de petites doses des gaz respectifs sur l'échantillon jusqu'à ce qu'une pression d'équilibre de 67 Pa environ soit atteinte. L'échantillon est alors dégazé pendant 30 min. à la même température et une seconde série d'adsorption analogue est réalisée jusqu'à ce qu'à une pression d'équilibre de 27 Pa environ soit atteinte. La différence entre les quantités adsorbées entre la première et la seconde adsorption (à 27 Pa) représente la quantité adsorbée irréversiblement des gaz respectifs qui fournit une estimation du nombre de sites forts respectivement acide ou base.

Dans certains cas, l'acidité ou la basicité du solide n'est révélée que si une faible adsorption d'eau ou d'alcool est préalablement réalisée sur le solide. Dans le cas d'une atmosphère parfaitement sèche, la surface probablement déshydratée est moins acide et/ou basique. L'adsorption préalable d'eau ou d'alcool peut être représentative des conditions opératoires de la réaction.

Le procédé de l'invention comporte deux phases successives : oxydation puis condensation par aldolisation, qui peuvent être effectuées en présence d'un catalyseur unique d'oxydation à base de molybdène. Ces deux phases sont conduites dans un système réactionnel comprenant un seul réacteur ou éventuellement deux réacteurs en cascade.

Dans le cas où l'ensemble réactionnel est constitué d'un seul réacteur, celui-ci comporte, soit un seul lit catalytique fixe constitué d'un unique catalyseur d'oxydation à base de molybdène ou d'un mélange physique des deux catalyseurs d'oxydation à base de molybdène et de condensation, soit de deux lits catalytiques superposés le premier (en amont) étant chargé d'un catalyseur d'oxydation et le second d'un catalyseur de condensation.

Dans la variante de réalisation avec deux réacteurs dans l'ensemble réactionnel, le premier réacteur sert à la réaction d'oxydation et le second réacteur sert à la réaction de condensation par aldolisation des composés issus directement du premier réacteur.

Dans cet ensemble réactionnel, les réactions sont conduites en phase gazeuse à une température comprise entre 200 et 400 °C et de préférence entre 250 et 350°C, sous une pression comprise entre 1 et 10 bars absolus et de préférence entre 1 et 5 bars absolus.

Les exemples suivants illustrent la présente invention sans toutefois en limiter la portée.

### Exemple 1 : Synthèse d'un catalyseur de condensation.

Le catalyseur de type Cs/Zr/SiO₂ est préparé à partir d'un gel de silice sous forme de particules de 315 à 500 microns ayant une pureté de 99,9%, une surface spécifique de 320 m²/g et un volume poreux de 0,9 cm³/g avec un diamètre de pore médian de 9 nm.

La silice est imprégnée par une solution de butoxyde de zirconium dans le butanol, filtration, et séchage en évaporateur tournant, puis dans un four à 120 °C pendant 2 heures. L'imprégnation et le séchage ont été répétés encore 2 fois de manière à obtenir un dépôt de 0,02 % en poids (1,2 g de zirconium pour 100 moles de silice). Le césium est alors lui aussi imprégné à partir d'une solution aqueuse de carbonate de césium, suivi d'un séchage, pour donner une teneur en césium d'environ 4 % pds (calculé en poids de métal). Le catalyseur a alors été calciné à 450 °C sous air pendant 3 heures. La surface spécifique du catalyseur ainsi préparé est de 147 m²/g.

### Exemple 2 : Synthèse d'un catalyseur de condensation

Le catalyseur Na/SiO₂ est préparé par imprégnation d'un gel de silice à gros pores, additionné de 12 % poids de silicate de sodium. Après imprégnation le solide est séché dans une étuve à 100°C pendant 10 h. puis calciné dans un four à 500°C pendant 3 heures. La surface spécifique du catalyseur préparé est de 122 m²/g.

### Exemple 3 : Préparation d'un catalyseur d'oxydation

On utilise un catalyseur molybdate de fer de type Mapco MS, disponible sous forme de pastilles, que l'on broie de manière à obtenir une poudre de granulométrie de 40 à 80 microns. Cette poudre est alors placée dans la trémie d'alimentation d'un enrobeur, et des billes de stéatite de marque CeramTec de diamètre moyen de 4,5 mm sont placées dans l'enrobeur. L'enrobeur (de type drageoir) est mis en rotation et on pulvérise sur les billes de stéatite une solution aqueuse de glycérol à 25 % pds, tout en ajoutant la poudre de molybdate de fer préalablement broyée de manière à ce qu'elle adhère aux billes de stéatite. Les billes sont ensuite séchées à 150 °C. La teneur en molybdate de fer dans le catalyseur supporté ainsi formé est de 20 % pds.

### Exemple 4 :

On reproduit l'enrobage de billes de stéatite comme décrit à l'exemple 3 avec les catalyseurs des exemples 1 et 2 après broyage sous forme de poudre de granulométrie comprise entre 40 et 80 microns.

### Exemple 5 : Synthèse de l'acroléine en présence d'un mélange de catalyseurs

On introduit dans un réacteur de 2 cm de diamètre interne 100 ml d'un mélange de catalyseur d'oxydation préparé à l'exemple 3, de type molybdate de fer supporté, et de catalyseur de condensation supportés de type Na/SiO₂ (dénommé Na dans le tableau 1) ou Cs/Zr/SiO₂ (dénommé Zr dans le tableau 1) préparés selon l'exemple 4.

Le débit d'injection de la charge gazeuse préchauffée à 120 °C, est de 33 l/minute, soit 2000 l/h. Le mélange réactionnel (éther/acétal/alcool) est injecté liquide dans un évaporateur dans lequel il rencontre les autres gaz, à l'exception du diméthyléther qui est injecté à l'état gazeux.

Le mélange gazeux de la charge comprend de 6 à 10 % vol d'oxygène, un mélange d'éther, acétal et/ou alcool dans les proportions en % vol indiquées dans le tableau 1, le complément à 100% étant l'azote.

Le mélange liquide est alimenté par une pompe, passe par un évaporateur avant de rejoindre le flux gazeux en amont du réacteur.

Le réacteur est placé dans un four électrique. La réaction est effectuée à une pression très légèrement supérieure à la pression atmosphérique et à une température variant de 300°C à 330°C.

Les effluents sont analysés par chromatographie avec détecteur FID (à ionisation de flamme) et avec un chromatographe équipé d'un couple méthaniseur-détecteur FID.

Les résultats obtenus sont donnés dans le tableau 1 ci-dessous.

Les rendements en produits sont calculés en tenant en compte du nombre de carbones dans les réactifs et produits.

Ainsi le rendement en acroléine est : 3*(nombre de moles d'acroléine détectées)/(nombre de moles de C1 entrant + 2*nombre de moles de C2 entrant).

C1 désigne les composés de la charge réactionnelle comportant des radicaux à un seul atome de carbone, et C2 désigne les composés de la charge réactionnelle comportant des radicaux à deux atomes de carbone.

**Tableau 1**

| Essai | Catalyseur ml | T,°C | Charge réactionnelle | | | | | | O₂ | Rendement Acroleine, |
|---|---|---|---|---|---|---|---|---|---|---|
| Essai | | | MeOH | EtOH | DME | DEE | DMM | DEM | | % mol |
| 1 | FeMo 75/ Na 25 | 325 | - | - | 2,5 | 2,5 | - | - | 7 | 45 |
| 2(comp) | FeMo 50 / Na 50 | 330 | 5 | 5 | - | - | - | - | 10 | 32 |
| 3 | FeMo 50 / Zr 50 | 320 | - | 5 | | | 1,5 | - | 9 | 42 |
| 4 | FeMo 25 / Zr 75 | 300 | | | 1 | | | 2,5 | 6 | 35 |

| | | | | | | | | | | |
|---|---|---|---|---|---|---|---|---|---|---|
| DME diméthyl éther ; DEE diéthyl éther ; DMM diméthoxyméthane ; DEM diéthoxyméthane | | | | | | | | | | |

### Exemple 6 : synthèse de l'acroléine en présence d'un catalyseur d'oxydation

Dans le réacteur utilisé dans l'exemple 5, on ne charge que le catalyseur de type molybdate de fer obtenu à l'exemple 3. La WH de la charge réactionnelle est de 15000 hr⁻¹.

On alimente le réacteur avec un mélange DMM/DEE dans un rapport molaire de 1,2, avec une pression partielle totale de réactifs de 6 %, et une pression partielle d'oxygène de 6 %, pour une pression totale de 1 atmosphère (absolue).

Les produits majoritaires détectés sont l'acroléine et l'acétaldéhyde. Le rendement en acroléine à 325 °C est de 25 %. L'acroléine a été produite en présence du seul catalyseur molybdate de fer supporté.

### Exemple 7

On procède comme à l'exemple précédent, mais en utilisant comme catalyseur d'oxydation, le catalyseur ACF-4 de Nippon Shokubai, qui est un catalyseur de type molybdate de bismuth utilisé notamment pour l'oxydation du propylène en acroléine. Le grade choisi est celui correspondant à une taille d'environ 5 mm. Comme pour les préparations précédentes, le catalyseur est broyé et enrobé sur des billes de stéatites.

La réaction est effectuée à 300°C, avec 100 ml de catalyseur. La charge comprend du dipropoxyméthane a une teneur de 2 % vol, et de l'oxygène à une teneur de 4 %. La VVH est 7 000 hr⁻¹.

On a obtenu de la méthacroléine avec un rendement de 12%.

## Revendications

1. Procédé de synthèse directe de (méth)acroléine comprenant i) la réaction d'un mélange réactif comprenant au moins un composé réactif, de l'oxygène et un gaz diluant non réactif, dans un ensemble réactionnel opéré en phase gazeuse à une température comprise entre 200°C et 400°C et sous une pression comprise entre 1 et 10 bars, en présence d'un catalyseur d'oxydation solide choisi parmi les catalyseurs à base de molybdène et éventuellement d'un catalyseur de condensation par aldolisation, puis ii) la récupération de l'effluent gazeux comprenant la (méth)acroléine formée en présence d'eau coproduite par la réaction,
ledit procédé étant **caractérisé**
- **en ce que** le(s) composé(s) réactif(s) est (sont) choisi(s) parmi les composés de formule (I) R₁-O-R₂ dans laquelle,
R₁ est H, ou un radical méthyl, éthyl ou propyl,
R₂ est un radical méthyl, éthyl, propyl ou un radical de formule CH₂-O-R₃, CH(CH₃)-O-R₃, CH(CH₂-CH₃)-O-R₃, avec R₃ est un radical méthyl, éthyl ou propyl,
avec R₁ et R₂ identiques ou différents, ou R₁ et R₃ identiques ou différents,
étant entendu que lorsque R₁ est H, R₂ est différent d'un radical méthyl, éthyl, propyl, à défaut de quoi le mélange réactif comprend au moins un autre composé de formule (I) avec R₁ différent de H,
- et **en ce que** le(s) composé(s) réactif(s) est (sont) choisi(s) de sorte que les radicaux R₁, R₂ et R₃ présents dans leur structure soient aptes à former de la (méth)acroléine par réaction d'oxydation couplée à une réaction d'aldolisation.

2. Procédé selon la revendication 1 **caractérisé en ce que** le composé réactif est choisi parmi le diméthyl éther, le diéthyl éther, le méthyl éthyl éther, le diméthoxy méthane, le diéthoxy méthane, le dipropoxy méthane, le 1,1-diméthoxy éthane ou le 1,1-diéthoxy éthane ; de préférence, le diméthyl éther, le diéthyl éther, le diméthoxy méthane, ou le 1,1-diméthoxy éthane.

3. Procédé selon la revendication 1 ou 2 **caractérisé en ce que** l'on utilise comme composé réactif ou d'association de composés réactifs pour synthétiser l'acroléine : diméthyl éther / diéthyl éther ; méthyl éthyl éther ; diméthyl éther / diéthoxy méthane ; diéthyl éther / diméthoxy méthane ; diméthoxy méthane / diéthoxy méthane ; diméthoxy méthane / diéthoxy éthane ; diméthyl éther / éthanol ; diéthyl éther / méthanol ; diméthoxy méthane / éthanol.

4. Procédé selon la revendication 1 ou 2 **caractérisé en ce que** l'on utilise comme composé réactif ou d'association de composés réactifs pour synthétiser la méthacroléine : le dipropoxyméthane, le dipropyl éther / méthanol ; le dipropyl éther / diméthyl éther.

5. Procédé selon l'une quelconque des revendications précédentes **caractérisé en ce que** le débit d'introduction des composés réactifs dans l'ensemble réactionnel est tel que leur teneur totale dans le mélange réactif est comprise entre 1% et 15%, de préférence entre 2% et 12%, et de manière plus préférée entre 2 et 6 % exprimée en volume.

6. Procédé selon l'une quelconque des revendications précédentes **caractérisé en ce que** le débit d'introduction de l'oxygène est tel que la teneur en oxygène du mélange réactif n'est pas supérieure à 15%, et de préférence inférieure à 10% exprimée en volume.

7. Procédé selon l'une quelconque des revendications précédentes **caractérisé en ce que** la réaction est conduite avec une vitesse volumique horaire comprise entre 2 000 et 40 000h⁻¹ et de préférence entre 5 000 et 25 000h⁻¹.

8. Procédé selon l'une quelconque des revendications précédentes **caractérisé en ce que** le catalyseur d'oxydation est représenté par la formule générale suivante.
**Aₐ X_{b} Mo**_{c} **Z_{d} O**ₑ (I)
dans laquelle
**A** est au moins un cation choisi parmi les éléments des Groupes 1 à 16 de la Classification Périodique des Eléments et les lanthanides, de préférence un cation d'un métal alcalin tel que Cs, Rb ou K,
**X** est P ou Si, et de préférence P
**Z** est au moins un élément choisi dans le groupe comprenant par W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce and Pb et de préférence Cu, Fe, Bi, Co, Ni, W, V, Cr, Sb, Mn, Ce,
**O** est l'oxygène
a, b, c et d sont des indices constitués de nombres entiers ou décimaux satisfaisant aux plages suivantes
0 ≤ a ≤ 9 et de préférence 0 < a ≤ 9
0 ≤ b ≤ 2 et de préférence 0,1 ≤ b ≤ 1,5
0 < c ≤ 12 et de préférence 5 < c ≤ 12
0 ≤ d ≤ 12 et de préférence 0 < d ≤ 4.
tels que a + b + d > 0
et e est un nombre déterminé par le degré total d'oxydation des éléments.

9. Procédé selon l'une quelconque des revendications précédentes **caractérisé en ce que** la réaction est conduite dans un réacteur unique avec un lit catalytique constitué d'un unique catalyseur d'oxydation à base de molybdène ou éventuellement d'un mélange physique d'un catalyseur d'oxydation à base de molybdène avec un catalyseur de condensation.

10. Procédé selon l'une quelconque des revendications 1 à 8 **caractérisé en ce que** la réaction est conduite dans un réacteur unique avec deux lits catalytiques superposés, le lit catalytique d'oxydation étant placé en amont du lit catalytique de condensation.

## Patentansprüche

1. Verfahren zur Direktsynthese von (Meth)acrolein, umfassend i) die Umsetzung einer Reaktionsmischung, die mindestens eine reaktive Verbindung, Sauerstoff und ein unreaktives Verdünnungsgas umfasst, in einem Reaktionsaufbau, der in der Gasphase bei einer Temperatur zwischen 200 °C und 400 °C und unter einem Druck zwischen 1 und 10 bar betrieben wird, in Gegenwart eines festen Oxidationskatalysators, der aus Katalysatoren auf Basis von Molybdän ausgewählt wird, und gegebenenfalls eines Aldolkondensationskatalysators und dann ii) die Gewinnung des gasförmigen Austragsstroms, der das in Gegenwart von bei der Reaktion als Nebenprodukt angefallenem Wasser gebildete (Meth)acrolein umfasst,
wobei das Verfahren **dadurch gekennzeichnet ist,**
- **dass** die reaktive Verbindung bzw. die reaktiven Verbindungen aus den Verbindungen der Formel (I) R₁-O-R₂ ausgewählt wird bzw. werden, wobei
R₁ für H oder einen Methyl-, Ethyl- oder Propylrest steht,
R₂ für einen Methyl-, Ethyl- oder Propylrest oder einen Rest der Formel CH₂-O-R₃, CH (CH₃) -O-R₃, CH(CH₂-CH₃)-O-R₃ steht, wobei R₃ für einen Methyl-, Ethyl- oder Propylrest steht,
wobei R₁ und R₂ gleich oder verschieden sein können oder R₁ und R₃ gleich oder verschieden sein können, mit der Maßgabe, dass dann, wenn R₁ für H steht, R₂ von einem Methyl-, Ethyl- oder Propylrest verschieden ist, oder stattdessen die Reaktionsmischung mindestens eine andere Verbindung der Formel (I), in der R₁ von H verschieden ist, umfasst,
- und **dass** die reaktive Verbindung bzw. die reaktiven Verbindungen so ausgewählt wird bzw. werden, dass die in ihrer Struktur vorliegenden Reste R₁, R₂ und R₃ zur Bildung von (Meth)acrolein durch eine mit einer Aldolkondensationsreaktion gekoppelten Oxidationsreaktion in der Lage sind.

2. Verfahren nach Anspruch 1, **dadurch gekennzeichnet, dass** die reaktive Verbindung aus Dimethylether, Diethylether, Methylethylether, Dimethoxymethan, Diethoxymethan, Dipropoxymethan, 1,1-Dimethoxyethan oder 1,1-Diethoxyethan, vorzugsweise Dimethylether, Diethylether, Dimethoxymethan oder 1,1-Dimethoxyethan ausgewählt wird.

3. Verfahren nach Anspruch 1 oder 2, **dadurch gekennzeichnet, dass** man als reaktive Verbindung oder Kombination von reaktiven Verbindungen zur Synthese von Acrolein Dimethylether / Diethylether; Methylethylether; Dimethylether / Diethoxymethan; Diethylether / Dimethoxymethan; Dimethoxymethan / Diethoxymethan; Dimethoxymethan / Diethoxyethan; Dimethylether / Ethanol; Diethylether / Methanol oder Dimethoxymethan / Ethanol verwendet.

4. Verfahren nach Anspruch 1 oder 2, **dadurch gekennzeichnet, dass** man als reaktive Verbindung oder Kombination von reaktiven Verbindungen zur Synthese von Methacrolein Dipropoxymethan; Dipropylether / Methanol; Dipropylether / Dimethylether verwendet.

5. Verfahren nach einem der vorhergehenden Ansprüche, **dadurch gekennzeichnet, dass** der Eintragsstrom der reaktiven Verbindungen in den Reaktionsaufbau so beschaffen ist, dass ihr Gesamtgehalt in der Reaktionsmischung zwischen 1 und 15 Vol.-%, vorzugsweise zwischen 2 und 12 Vol.-% und weiter bevorzugt zwischen 2 und 6 Vol.-% liegt.

6. Verfahren nach einem der vorhergehenden Ansprüche, **dadurch gekennzeichnet, dass** der Eintragsstrom des Sauerstoffs so beschaffen ist, dass der Sauerstoffgehalt der Reaktionsmischung nicht über 15 Vol.-% und vorzugsweise unter 10 Vol.-% liegt.

7. Verfahren nach einem der vorhergehenden Ansprüche, **dadurch gekennzeichnet, dass** die Reaktion mit einer Katalysatorbelastung zwischen 2000 und 40.000 h⁻¹ und vorzugsweise zwischen 5000 und 25.000 h⁻¹ durchgeführt wird.

8. Verfahren nach einem der vorhergehenden Ansprüche, **dadurch gekennzeichnet, dass** der Oxidationskatalysator durch die folgende allgemeine Formel wiedergegeben wird:
**A**ₐ **X**_{b} **Mo**_{c} **Z**_{d} **O**ₑ (I),
wobei
**A** für mindestens ein Kation, das aus den Elementen der Gruppen 1 bis 16 des Periodensystems der Elemente und den Lanthaniden ausgewählt ist, vorzugsweise ein Kation eines Alkalimetalls wie Cs, Rb oder K, steht,
**X** für P oder Si und vorzugsweise P steht,
**Z** für mindestens ein Element aus der Gruppe umfassend W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce und Pb und vorzugsweise Cu, Fe, Bi, Co, Ni, W, V, Cr, Sb, Mn und Ce ausgewählt ist,
**O** für Sauerstoff steht,
a, b, c und d für Indices stehen, die aus ganzen Zahlen oder Dezimalzahlen bestehen, die den folgenden Bereichen entsprechen:
0 ≤ a ≤ 9 und vorzugsweise 0 < a ≤ 9,
0 ≤ b ≤ 2 und vorzugsweise 0,1 ≤ b ≤ 1,5,
0 < c ≤ 12 und vorzugsweise 5 < c ≤ 12,
0 ≤ d ≤ 12 und vorzugsweise 0 < d ≤ 4,
derart, dass a + b + d > 0,
und e für eine durch die Gesamtoxidationsstufe der Elemente bestimmte Zahl steht.

9. Verfahren nach einem der vorhergehenden Ansprüche, **dadurch gekennzeichnet, dass** die Reaktion in einem einzigen Reaktor mit einer katalytischen Schüttung, die aus einem einzigen Oxidationskatalysator auf Basis von Molybdän oder gegebenenfalls einer physikalischen Mischung eines Oxidationskatalysators auf Basis von Molybdän mit einem Kondensationskatalysator besteht, durchgeführt wird.

10. Verfahren nach einem der Ansprüche 1 bis 8, **dadurch gekennzeichnet, dass** die Reaktion in einem einzigen Reaktor mit zwei übereinander angeordneten katalytischen Schüttungen durchgeführt wird, wobei die katalytische Schüttung zur Oxidation stromaufwärts der katalytischen Schüttung zur Kondensation angeordnet ist.

## Claims

1. A process for direct synthesis of (meth)acrolein comprising i) reacting a reactive mixture comprising at least one reactive compound, oxygen and a non-reactive diluent gas, in a reaction assembly operated in the gas phase at a temperature of between 200°C and 400°C and at a pressure of between 1 and 10 bar, in the presence of a solid oxidation catalyst chosen from molybdenum-based catalysts and optionally of an aldol condensation catalyst, then ii) recovering the gas effluent comprising the (meth)acrolein formed in the presence of water coproduced by the reaction,
said process being **characterized**
- **in that** the reaction compound(s) is (are) chosen from the compounds of formula (I) R₁-O-R₂ in which,
R₁ is H or a methyl, ethyl or propyl radical,
R₂ is a methyl, ethyl or propyl radical or a radical of formula CH₂-O-R₃, CH(CH₃)-O-R₃ or CH(CH₂-CH₃)-O-R₃, with R₃ being a methyl, ethyl or propyl radical,
with R₁ and R₂ identical or different, or R₁ and R₃ identical or different,
it being understood that, when R₁ is H, R₂ is other than a methyl, ethyl or propyl radical, failing which the reactive mixture comprises at least one other compound of formula (I) with R₁ other than H,
- and **in that** the reactive compound(s) is (are) chosen such that the R₁, R₂ and R₃ radicals present in their structure are capable of forming (meth)acrolein by means of an oxidation reaction coupled to an aldol condensation reaction.

2. The process as claimed in claim 1, **characterized in that** the reactive compound is chosen from dimethyl ether, diethyl ether, methyl ethyl ether, dimethoxymethane, diethoxymethane, dipropoxymethane, 1,1-dimethoxyethane or 1,1-diethoxyethane; preferably dimethyl ether, diethyl ether, dimethoxymethane or 1,1-dimethoxyethane.

3. The process as claimed in claim 1 or 2, **characterized in that** use is made, as reactive compound or a combination of reactive compounds for synthesizing acrolein, of: dimethyl ether / diethyl ether; methyl ethyl ether; dimethyl ether / diethoxymethane; diethyl ether / dimethoxymethane; dimethoxymethane / diethoxymethane; dimethoxymethane / diethoxyethane; dimethyl ether / ethanol; diethyl ether / methanol; and dimethoxymethane / ethanol.

4. The process as claimed in claim 1 or 2, **characterized in that** use is made, as reactive compound or a combination of reactive compounds for synthesizing methacrolein, of: dipropoxymethane; dipropyl ether / methanol; and dipropyl ether / dimethyl ether.

5. The process as claimed in any one of the preceding claims, **characterized in that** the flow rate for introduction of the reactive compounds into the reaction assembly is such that their total content in the reactive mixture is between 1% and 15%, preferably between 2% and 12%, and more preferably between 2% and 6% expressed by volume.

6. The process as claimed in any one of the preceding claims, **characterized in that** the oxygen introduction flow rate is such that the oxygen content of the reactive mixture is not greater than 15%, and preferably less than 10% expressed by volume.

7. The process as claimed in any one of the preceding claims, **characterized in that** the reaction is carried out with an hourly space velocity of between 2000 and 40 000 h⁻¹ and preferably between 5000 and 25 000 h⁻¹.

8. The process as claimed in any one of the preceding claims, **characterized in that** the oxidation catalyst is represented by the following general formula:
**A**ₐ **X**_{b} **Mo**_{c} **Z**_{d} **O**ₑ (I)
in which
**A** is at least one cation chosen from the elements of groups 1 to 16 of the Periodic Table of Elements and lanthanides, preferably a cation of an alkali metal such as Cs, Rb or K,
**X** is P or Si, and preferably P,
**Z** is at least one element chosen from the group comprising W, Ti, Zr, V, Nb, Ta, Cr, Mn, Fe, Co, Ni, Cu, Zn, Ga, In, TI, Sn, Ag, As, Ge, B, Bi, La, Ba, Sb, Te, Ce and Pb and preferably Cu, Fe, Bi, Co, Ni, W, V, Cr, Sb, Mn and Ce,
**O** is oxygen,
a, b, c and d are indices consisting of whole or decimal numbers which satisfy the following ranges
0 ≤ a ≤ 9 and preferably 0 < a ≤ 9
0 ≤ b ≤ 2 and preferably 0.1 ≤ b ≤ 1.5
0 < c ≤ 12 and preferably 5 < c ≤ 12
0 ≤ d ≤ 12 and preferably 0 < d ≤ 4
such that a + b + d > 0
and e is a number determined by the total degree of oxidation of the elements.

9. The process as claimed in any one of the preceding claims, **characterized in that** the reaction is carried out in a single reactor with a catalytic bed consisting of a single molybdenum-based oxidation catalyst or optionally of a physical mixture of a molybdenum-based oxidation catalyst with a condensation catalyst.

10. The process as claimed in any one of claims 1 to 8, **characterized in that** the reaction is carried out in a single reactor with two superimposed catalytic beds, the oxidation catalytic bed being placed upstream of the condensation catalytic bed.
